# EUROPÄISCHE PATENTANMELDUNG

(11) **EP 2 248 512 A2**
(43) Veröffentlichungstag der Anmeldung: **10.11.2010**
(21) Anmeldenummer: 10008258.5
(22) Anmeldetag: 14.11.2008
(51) Int. Cl.: A61K 8/44, A61Q 19/10

(54) **Zusammensetzungen enthaltend Acylglycinate**

(30) Priorität: 20.11.2007 DE 102007055265
(62) Teilanmeldung aus: 08852108.3
(71) Anmelder: Clariant Finance (BVI) Limited, Road Town, Tortola (VG)
(72) Erfinder: Klug, Peter, 63762 Großostheim (DE); Scherl, Franz-Xaver, 84508 Burgkirchen (DE)
(74) Vertreter: Paczkowski, Marcus

(57) **Zusammenfassung**

Es werden Zusammensetzungen beschrieben enthaltend
a) ein oder mehrere Acylglycinate der Formel (I) 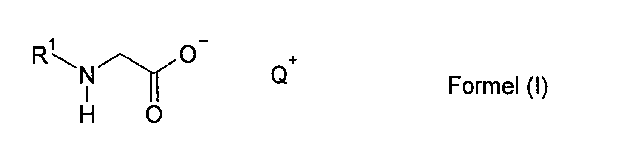 worin
R¹ für eine lineare oder verzweigte, gesättigte Alkanoylgruppe mit 6 bis 30 Kohlenstoffatomen oder für eine lineare oder verzweigte, ein- oder mehrfach ungesättigte Alkenoylgruppe mit 6 bis 30Kohlenstoffatomen, steht, und Q⁺ für ein Kation ausgewählt aus den Alkalimetallkationen Li⁺, Na⁺ und K⁺ steht,
in Mengen von 21,0 - 28,0 Gew.-%, bezogen auf die gesamte Zusammensetzung,
und wobei der Anteil an Acylglycinaten der Formel (I) enthaltend Acylgruppen R¹ mit 18 oder mehr Kohlenstoffatomen, bezogen auf die Gesamtmenge an
Acylglycinaten der Formel (I), kleiner als 2,0 Gew.-% ist,
b) ein oder mehrere Substanzen Q⁺Cl⁻, worin Q⁺ die Bedeutung von Q⁺ aus Formel (I) hat, in Mengen größer oder gleich 1,0 Gew.-%, bezogen auf die gesamte Zusammensetzung,
c) ein oder mehrere Fettsäuresalze der Formel (II) 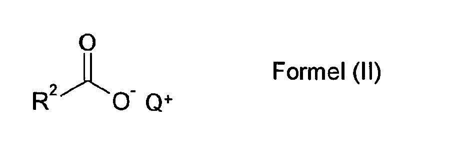 worin
R²CO die Bedeutung von R¹ aus Formel (I) und Q⁺ die Bedeutung von Q⁺ aus Formel (I) hat,
in Mengen kleiner als 2,0 Gew.-%, bezogen auf die gesamte
Zusammensetzung, und
d) Wasser,
e) aber keine organischen Lösungsmittel.

Die Zusammensetzungen können in vorteilhafter Weise zur Herstellung von kosmetischen Zubereitungen sowie als Tensid in kosmetischen Zubereitungen verwendet werden.

## Beschreibung

Die Erfindung betrifft ein Verfahren zur Herstellung von Acylglycinaten sowie Zusammensetzungen enthaltend Acylglycinate.

### Acylglycinate der Formel (la)

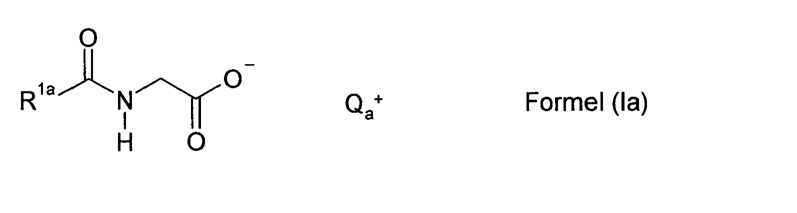

worin
R^{1a}-C(O) für eine lineare oder verzweigte, gesättigte Alkanoylgruppe mit 6 bis 30, vorzugsweise 8 bis 22, besonders bevorzugt 8 bis 18 Kohlenstoffatomen, oder für eine lineare oder verzweigte, ein- oder mehrfach ungesättigte Alkenoylgruppe mit 6 bis 30, vorzugsweise 8 bis 22 und besonders bevorzugt 12 bis 18 Kohlenstoffatomen steht, und
Qₐ⁺ für ein Kation steht, ausgewählt aus den Alkalimetallen Li⁺, Na⁺, K⁺, den Erdalkalimetallen Mg⁺⁺, Ca⁺⁺, aber auch für Al⁺⁺⁺ und/oder NH₄⁺, ein Monoalkylammonium-, Dialkylammonium-, Trialkylammonium- und/oder Tetraalkylammoniumion, wobei es sich bei den Alkylsubstituenten der Amine unabhängig voneinander um (C₁ - C₂₂)-Alkylreste oder (C₂ - C₁₀)-Hydroxyalkylreste handeln kann,
sind Tenside, die besonders in der Kosmetik für Gesichtsreinigungsrezepturen in Asien in Hautreinigungsprodukten geschätzt werden. Dies gilt insbesondere für Natrium- und Kaliumcocoylglycinat.

Die Tenside, insbesondere das Natrium- und das Kaliumcocoylglycinat, schäumen in leicht alkalischer Lösung exzellent und erzeugen ein angenehmes, nicht öliges Hautgefühl.

Im Gegensatz zu den entsprechenden N-Methylglycinderivaten, den sogenannten Sarkosinaten, weisen Glycinate bei der Herstellung jedoch problematische Eigenschaften auf. Wie in JP 8053693 beschrieben, lassen sich Acylglycinate in Wasser ohne zusätzliche Lösungsmittel nur in Reinheiten von knapp über 90 % erhalten und bilden bei der Schotten-Baumann-Reaktion sehr hochviskose Reaktionslösungen. Durch diese hohe Viskosität wird die Hydrolyse des zur Herstellung verwendeten Fettsäurechlorids begünstigt, die zu verminderter Reinheit des Acylglycinats führt. Die JP 8053693 schlägt daher bei der Herstellung von Acylglycinaten die Zugabe von Alkoholen wie Isopropanol, Isobutanol oder tert.-Butanol vor.

Diese Vorgehensweise ist jedoch wegen des Geruchs der Alkohole nicht vorteilhaft, weshalb die Alkohole aus der Reaktionsmischung auch nach Ansäuern und Phasentrennung wieder entfernt werden und das gewünschte Alkanoylglycinat nach Neutralisation meist als salzarme Qualität erhalten wird. Dieses Verfahren ist aufwendig und verursacht kochsalzhaltige Abwässer, die entsorgt werden müssen.

Es bestand daher die Aufgabe, ein Verfahren zur Herstellung von Acylglycinaten bzw. von Zusammensetzungen enthaltend Acylglycinate zur Verfügung zu stellen, das die oben genannten Nachteile nicht aufweist oder diese Nachteile zumindest abschwächt und insbesondere den Vorteil aufweist, dass es ohne Einsatz von organischen Lösemitteln erfolgen kann und Acylglycinate von hoher Reinheit liefert. Das Verfahren sollte auch die direkte Herstellung von Zusammensetzungen mit hohem Aktivgehalt an Acylglycinat und mit niedrigen Viskositäten ermöglichen.

Überraschenderweise wurde gefunden, dass diese Aufgabe gelöst wird, wenn die Acylglycinate durch Umsetzung von Glycin mit Fettsäurechlorid in Wasser und in Gegenwart einer basischen Alkaliverbindung, aber in Abwesenheit von organischen Lösungsmitteln, hergestellt werden, die Herstellung der Acylglycinate bei einer Temperatur von 30 - 35°C durchgeführt wird, und der Anteil an Fettsäurechlorid enthaltend Acylgruppen mit 18 oder mehr Kohlenstoffatomen, bezogen auf die Gesamtmenge an eingesetztem Fettsäurechlorid, kleiner als 2,0 Gew.-% ist.

Gegenstand der Erfindung ist daher ein Verfahren zur Herstellung von Acylglycinaten der Formel (I) 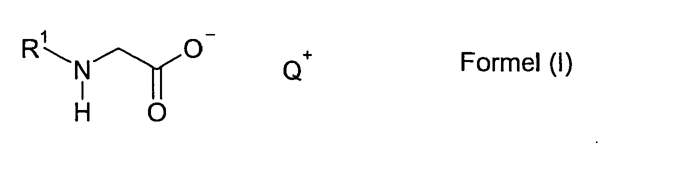 worin
R¹ für eine lineare oder verzweigte, gesättigte Alkanoylgruppe mit 6 bis 30, vorzugsweise 8 bis 22 und besonders bevorzugt 8 bis 18 Kohlenstoffatomen, oder für eine lineare oder verzweigte, ein- oder mehrfach ungesättigte Alkenoylgruppe mit 6 bis 30, vorzugsweise 8 bis 22 und besonders bevorzugt 12 bis 18 Kohlenstoffatomen, steht, und
Q⁺ für ein Kation ausgewählt aus den Alkalimetallkationen Li⁺, Na⁺ und K⁺, vorzugsweise ausgewählt aus Na⁺ und K⁺ und besonders bevorzugt für Na⁺, steht,
**dadurch gekennzeichnet, dass** Glycin mit Fettsäurechlorid R¹Cl, wobei R¹ die in Formel (I) angegebene Bedeutung besitzt, in Wasser und in Gegenwart einer basischen Alkaliverbindung, die Kationen Q⁺ ausgewählt aus Li⁺, Na⁺ und K⁺, vorzugsweise ausgewählt aus Na⁺ und K⁺ und besonders bevorzugt Na⁺, liefert, aber in Abwesenheit von organischen Lösungsmitteln, bei 30 - 35 °C umgesetzt wird, und der Anteil an Fettsäurechlorid R¹Cl enthaltend Acylgruppen R¹ mit 18 oder mehr Kohlenstoffatomen, bezogen auf die Gesamtmenge an eingesetztem Fettsäurechlorid, kleiner als 2,0 Gew.-% ist.

Im Rahmen der vorliegenden Erfindung werden die linearen oder verzweigten, gesättigten Alkanoylgruppen R¹ aus Formel (I) und die linearen oder verzweigten, ein- oder mehrfach ungesättigten Alkenoylgruppen R¹ aus Formel (I) zusammen auch als "Acylgruppen" bezeichnet.

Die im erfindungsgemäßen Verfahren verwendeten Fettsäurechloride mit niedrigem Anteil an langkettigen Acylgruppen können nach dem Fachmann bekannten Methoden erhalten werden, z. B. durch Destillation aus gewöhnlichen Fettsäurechloriden.

Als basische Alkaliverbindungen werden vorzugsweise Carbonate oder Hydroxide, vorzugsweise Hydroxide, der Alkalimetallkationen Li⁺, Na⁺ oder K⁺ oder Mischungen davon eingesetzt. Besonders bevorzugt sind NaOH und/oder KOH, insbesondere bevorzugt ist NaOH.

Das erfindungsgemäße Verfahren wird vorzugsweise bei einem pH-Wert von 9 bis 13, besonders bevorzugt 12 bis 13, durchgeführt.

Weiterhin bevorzugt wird das erfindungsgemäße Verfahren derart durchgeführt, dass Glycin und Fettsäurechlorid in equimolaren Mengen eingesetzt werden. Besonders bevorzugt wird das Fettsäurechlorid bezogen auf Glycin in leichtem Unterschuss eingesetzt. Das molare Verhältnis von Glycin zu Fettsäurechlorid R¹Cl ist insbesondere bevorzugt von 1,1 : 1,0 bis 1,0 : 1,0 und außerordentlich bevorzugt von 1,05 : 1,0 bis 1,0 : 1,0.

In einer weiteren bevorzugten Ausführungsform des erfindungsgemäßen Verfahrens werden Fettsäurechloride R¹Cl enthaltend Acylgruppen R¹ mit 8 bis 18 Kohlenstoffatomen, wobei der Anteil an Fettsäurechlorid R¹Cl enthaltend Acylgruppen R¹ mit 18 Kohlenstoffatomen, bezogen auf die Gesamtmenge an eingesetztem Fettsäurechlorid R¹Cl, kleiner als 2,0 Gew.-% ist, in Gegenwart einer basischen Alkaliverbindung, die Kationen Q⁺ ausgewählt aus Li⁺, Na⁺ und K⁺, vorzugsweise ausgewählt aus Na⁺ und K⁺ und besonders bevorzugt Na⁺, liefert, umgesetzt. Bei den basischen Alkaliverbindungen handelt es sich vorzugsweise um Carbonate oder Hydroxide, vorzugsweise um Hydroxide, der Alkalimetallkationen Li⁺, Na⁺ oder K⁺ oder Mischungen davon, besonders bevorzugt um NaOH oder KOH und insbesondere bevorzugt um NaOH.

In einer weiteren bevorzugten Ausführungsform des erfindungsgemäßen Verfahrens werden C₈₋₁₈-Fettsäurechloride, vorzugsweise Cocoylchloride, die einen Anteil an Fettsäurechlorid mit C₁₈-Acylgruppen kleiner 2,0 Gew.-%, bezogen auf die Gesamtmenge an eingesetztem Fettsäurechlorid, enthalten, in Gegenwart einer basischen Alkaliverbindung, die Kationen Q⁺ ausgewählt aus Li⁺, Na⁺ und K⁺, vorzugsweise ausgewählt aus Na⁺ und K⁺ und besonders bevorzugt Na⁺, liefert, umgesetzt. Bei den basischen Alkaliverbindungen handelt es sich vorzugsweise um Carbonate oder Hydroxide, vorzugsweise um Hydroxide, der Alkalimetallkationen Li⁺, Na⁺ oder K⁺ oder Mischungen davon, besonders bevorzugt um NaOH oder KOH und insbesondere bevorzugt um NaOH.

In einer besonders bevorzugten Ausführungsform des erfindungsgemäßen Verfahrens werden Cocoylchloride mit C₈₋₁₈-Acylgruppen, und wobei der Anteil an Fettsäurechloriden mit C₁₈-Acylgruppen, bezogen auf die Gesamtmenge an eingesetztem Cocoylchlorid, kleiner als 2,0 und bevorzugt kleiner als 1,0 Gew.-% ist, in Gegenwart einer basischen Alkaliverbindung, die Na⁺-Kationen liefert, umgesetzt. Bei den basischen Alkaliverbindungen handelt es sich vorzugsweise um Na₂CO₃ oder NaOH und besonders bevorzugt um NaOH.

In einer insbesondere bevorzugten Ausführungsform des erfindungsgemäßen Verfahrens werden Cocoylchloride mit C₈-₁₈-Acylgruppen, und wobei der Anteil an Fettsäurechloriden mit C₈- und C₁₀-Acylgruppen zusammen größer als 5,0 Gew.-%, bevorzugt von 10,0 bis 14,0 Gew.-%, der Anteil an Fettsäurechloriden mit C₁₂-Acylgruppen von 50,0 bis 72,0 Gew.-%, und der Anteil an Fettsäurechloriden mit C₁₈-Acylgruppen kleiner als 2,0 und bevorzugt kleiner als 1,0 Gew.-%, jeweils bezogen auf die Gesamtmenge an eingesetztem Cocoylchlorid, ist, in Gegenwart einer basischen Alkaliverbindung, die Na⁺-Kationen liefert, umgesetzt. Bei den basischen Alkaliverbindungen handelt es sich vorzugsweise um Na₂CO₃ oder NaOH und besonders bevorzugt um NaOH.

In einer weiteren bevorzugten Ausführungsform des erfindungsgemäßen Verfahrens werden Fettsäurechloride R¹Cl, worin R¹ für eine Acylgruppe mit 12 Kohlenstoffatomen steht, in Gegenwart einer basischen Alkaliverbindung, die Kationen Q⁺ ausgewählt aus Li⁺, Na⁺ und K⁺, vorzugsweise ausgewählt aus Na⁺ und K⁺ und besonders bevorzugt Na⁺, liefert, umgesetzt. Bei den basischen Alkaliverbindungen handelt es sich vorzugsweise um Carbonate oder Hydroxide, vorzugsweise um Hydroxide, der Alkalimetallkationen Li⁺, Na⁺oder K⁺ oder Mischungen davon, besonders bevorzugt um NaOH oder KOH und insbesondere bevorzugt um NaOH. Bei den genannten Fettsäurechloriden handelt es sich vorzugsweise um Lauroylchlorid.

In einer weiteren bevorzugten Ausführungsform des erfindungsgemäßen Verfahrens werden Fettsäurechloride R¹Cl, worin R¹ für eine Acylgruppe mit 12 Kohlenstoffatomen steht, und zusätzlich Fettsäurechloride R¹Cl, worin R¹ für eine Acylgruppe mit 14 Kohlenstoffatomen steht, in Gegenwart einer basischen Alkaliverbindung, die Kationen Q⁺ ausgewählt aus Li⁺, Na⁺ und K⁺, vorzugsweise ausgewählt aus Na⁺ und K⁺ und besonders bevorzugt Na⁺, liefert, umgesetzt. Bei den basischen Alkaliverbindungen handelt es sich vorzugsweise um Carbonate oder Hydroxide, vorzugsweise um Hydroxide, der Alkalimetallkationen Li⁺, Na⁺ oder K⁺ oder Mischungen davon, besonders bevorzugt um NaOH oder KOH und insbesondere bevorzugt um NaOH.

Das erfindungsgemäße Verfahren wird vorzugsweise derart ausgeführt, dass Glycin in Wasser in Gegenwart der basischen Alkaliverbindung wie beispielsweise NaOH vorgelegt wird und das Fettsäurechlorid bei 30 bis 35 °C zugegeben wird. Die Zugabe des Fettsäurechlorids erfolgt dabei vorzugsweise langsam unter Rühren.

Durch das erfindungsgemäße Verfahren können höher konzentrierte, salzhaltige Glycinatlösungen mit niedrigem Gehalt an Nebenprodukten (wie beispielsweise

Fettsäuresalz) hergestellt werden, die niedrigviskos und dementsprechend einfach zu handhaben sind und darüber hinaus kosteneffektiv sind, da kein Separationsschritt durchgeführt werden muss. Außerdem wird dabei kein organisches Reaktionslösungsmittel benötigt, das wieder abgetrennt und gegebenenfalls entsorgt werden muss.

Weiterer Gegenstand der Erfindung sind daher Zusammensetzungen enthaltend
a) ein oder mehrere Acylglycinate der Formel (I) 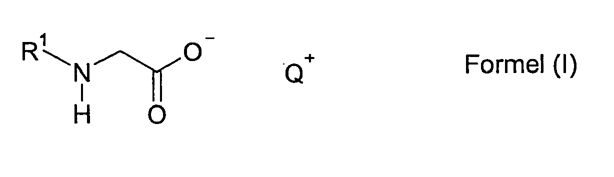 worin
   R¹ für eine lineare oder verzweigte, gesättigte Alkanoylgruppe mit 6 bis 30, vorzugsweise 8 bis 22 und besonders bevorzugt 8 bis 18 Kohlenstoffatomen, oder für eine lineare oder verzweigte, ein- oder mehrfach ungesättigte Alkenoylgruppe mit 6 bis 30, vorzugsweise 8 bis 22 und besonders bevorzugt 12 bis 18 Kohlenstoffatomen, steht, und
   Q⁺ für ein Kation ausgewählt aus den Alkalimetallkationen Li⁺, Na⁺ und K⁺, vorzugsweise ausgewählt aus Na⁺ und K⁺ und besonders bevorzugt für Na⁺, steht,
   in Mengen von 21,0 - 28,0 Gew.-%, bezogen auf die gesamte Zusammensetzung,
   und wobei der Anteil an Acylglycinaten der Formel (I) enthaltend Acylgruppen R¹ mit 18 oder mehr Kohlenstoffatomen, bezogen auf die Gesamtmenge an Acylglycinaten der Formel (I), kleiner als 2,0, bevorzugt kleiner als 1,8, Gew.-% ist,
b) ein oder mehrere Substanzen Q⁺Cl⁻, worin Q⁺ die Bedeutung von Q⁺ aus Formel (I) hat, in Mengen größer oder gleich 1,0 Gew.-%, bezogen auf die gesamte Zusammensetzung,
c) ein oder mehrere Fettsäuresalze der Formel (II) 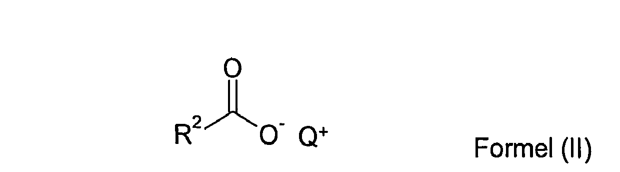 worin
   R²CO die Bedeutung von R¹ aus Formel (I) und
   Q⁺ die Bedeutung von Q⁺ aus Formel (I) hat,
   in Mengen kleiner als 2,0 Gew.-%, vorzugsweise in Mengen größer als 0,01 Gew.-% und kleiner als 2,0 Gew.-% und besonders bevorzugt in Mengen größer als 0,1 Gew.-% und kleiner als 2,0 Gew.-%, bezogen auf die gesamte Zusammensetzung, und
d) Wasser,
e) aber keine organischen Lösungsmittel.
   Q⁺Cl⁻ wird auch als QCI bezeichnet, z.B. Na⁺Cl⁻ als NaCl.

In den Verbindungen der Formeln (I) und (II) sowie in Q⁺Cl⁻ ist Q⁺ ausgewählt aus Li⁺, Na⁺ und K⁺, vorzugsweise ausgewählt aus Na⁺ und K⁺ und besonders bevorzugt steht Q⁺ für Na⁺.

In einer bevorzugten Ausführungsform der Erfindung enthalten die erfindungsgemäßen Zusammensetzungen als Komponente a) ein oder mehrere Acylglycinate der Formel (I), worin R¹ für eine Acylgruppe mit 8 bis 18 Kohlenstoffatomen steht, und Q⁺ für ein Kation ausgewählt aus Li⁺, Na⁺ und K⁺, vorzugsweise ausgewählt aus Na⁺ und K⁺ und besonders bevorzugt für Na⁺, steht, in Mengen von 21,0 - 28,0 Gew.-% und vorzugsweise in Mengen von 23,0 - 27,0 Gew.-%, bezogen auf die gesamte Zusammensetzung, und wobei der Anteil an Acylglycinaten der Formel (I) enthaltend Acylgruppen R¹ mit 18 Kohlenstoffatomen, bezogen auf die Gesamtmenge an Acylglycinaten der Formel (I), kleiner als 2,0, bevorzugt kleiner als 1,8, Gew.-% ist.

Besonders bevorzugt sind Zusammensetzungen enthaltend Natriumcocoylglycinat, wobei der Cocoylschnitt nur geringe Mengen an C₁₈-Acylgruppen aufweist.

In einer besonders bevorzugten Ausführungsform der Erfindung enthalten die erfindungsgemäßen Zusammensetzungen als Komponente a) Natriumcocoylglycinat mit C₈-₁₈-Acylgruppen in Mengen von 21,0 - 28,0 Gew.-% und vorzugsweise in Mengen von 23,0 - 27,0 Gew.-%, bezogen auf die gesamte Zusammensetzung, und wobei der Anteil an Acylglycinaten der Formel (I) mit C₁₈-Acylgruppen kleiner als 2,0, bevorzugt kleiner als 1,8, und besonders bevorzugt kleiner als 1,0 Gew.-%, bezogen auf die Gesamtmenge an Natriumcocoylglycinat, ist. In dieser Ausführungsform ist Q⁺ in Q⁺Cl⁻ und in dem Fettsäuresalz der Formel (II) Na⁺.

In einer insbesondere bevorzugten Ausführungsform der Erfindung enthalten die erfindungsgemäßen Zusammensetzungen als Komponente a) Natriumcocoylglycinat mit C₈-₁₈-Acylgruppen in Mengen von 21,0 - 28,0 Gew.-% und vorzugsweise in Mengen von 23,0 - 27,0 Gew.-%, bezogen auf die gesamte Zusammensetzung, und wobei der Anteil an Acylglycinaten der Formel (I) mit C₈- und C₁₀-Acylgruppen zusammen größer als 5,0 Gew.-%, bevorzugt von 10,0 bis 14,0 Gew.-%, der Anteil an Acylglycinaten der Formel (I) mit C₁₂-Acylgruppen von 50,0 bis 72,0 Gew.-%, und der Anteil an Acylglycinaten der Formel (I) mit C₁₈-Acylgruppen kleiner als 2,0, bevorzugt kleiner als 1,8, und besonders bevorzugt kleiner als 1,0 Gew.-%, jeweils bezogen auf die Gesamtmenge an Natriumcocoylglycinat, ist. In dieser Ausführungsform ist Q⁺ in Q⁺Cl⁻ und in dem Fettsäuresalz der Formel (II) Na⁺.

In einer außerordentlich bevorzugten Ausführungsform der Erfindung ist die Reinheit der in den erfindungsgemäßen Zusammensetzungen enthaltenen Natriumcocoylglycinate 97 % oder größer. Diese Reinheit ist auf die Summe an Fettsäuresalz der Formel (II) und Acylglycinat der Formel (I) bezogen. Sie berechnet sich nach der Formel "Reinheit der Natriumcocoylglycinate = [Menge an Natriumcocoylglycinat : (Menge an Natriumcocoylglycinat + Menge an Fettsäuresalz)]".

In einer weiteren bevorzugten Ausführungsform der Erfindung enthalten die erfindungsgemäßen Zusammensetzungen als Komponente a) ein oder mehrere Acylglycinate der Formel (I), worin R¹ für eine Acylgruppe mit 12 Kohlenstoffatomen steht, und Q⁺ für ein Kation ausgewählt aus Li⁺, Na⁺ und K⁺, vorzugsweise ausgewählt aus Na⁺ und K⁺ und besonders bevorzugt für Na⁺, steht, in Mengen von 21,0 - 28,0 Gew.-% und vorzugsweise in Mengen von 23,0 - 27,0 Gew.-%, bezogen auf die gesamte Zusammensetzung. Unter diesen Acylglycinaten der Formel (I) sind wiederum diejenigen bevorzugt, in denen sich die Acylgruppe von Lauroylchlorid ableitet.

In einer weiteren bevorzugten Ausführungsform der Erfindung enthalten die erfindungsgemäßen Zusammensetzungen als Komponente a) ein oder mehrere Acylglycinate der Formel (I), worin R¹ für eine Acylgruppe mit 12 Kohlenstoffatomen steht, und zusätzlich ein oder mehrere Acylglycinate der Formel (I), worin R¹ für eine Acylgruppe mit 14 Kohlenstoffatomen steht, und Q⁺ jeweils für ein Kation ausgewählt aus Li⁺, Na⁺ und K⁺, vorzugsweise ausgewählt aus Na⁺ und K⁺ und besonders bevorzugt für Na⁺, steht, zusammen in Mengen von 21,0 - 28,0 Gew.-% und vorzugsweise in Mengen von 23,0 - 27,0 Gew.-%, bezogen auf die gesamte Zusammensetzung.

Die erfindungsgemäßen Zusammensetzungen können beispielsweise vorteilhaft für kosmetische Anwendungen verwendet werden.

Für die kosmetischen Anwendungen sehr vorteilhaft ist, dass die erfindungsgemäßen Zusammensetzungen Q⁺Cl⁻ in Mengen von 1,0 bis 8,0 Gew.-% enthalten, so dass eine gesonderte Zugabe von Q⁺Cl⁻ als Viskositätsregulierer in den Endformulierungen entfallen kann.

In einer weiteren bevorzugten Ausführungsform der Erfindung enthalten die erfindungsgemäßen Zusammensetzungen daher 1,0 bis 8,0, bevorzugt 2,0 bis 7,0 und besonders bevorzugt 4,0 bis 6,0 Gew.-% Q⁺Cl⁻, bezogen auf die gesamte Zusammensetzung.

Ein weiterer Vorteil der erfindungsgemäßen Zusammensetzungen ist, dass diese nur geringe Mengen an Fettsäuresalz enthalten.

In einer weiteren bevorzugten Ausführungsform der Erfindung enthalten die erfindungsgemäßen Zusammensetzungen daher weniger als 1,8, bevorzugt weniger als 1,5 und besonders bevorzugt weniger als 1,0 Gew.-% an Fettsäuresalz der Formel (II), bezogen auf die gesamte Zusammensetzung. Dabei ist die untere Grenze vorzugsweise jeweils größer 0,01 Gew.-% und besonders bevorzugt jeweils größer 0,1 Gew.-%, bezogen auf die gesamte Zusammensetzung.

Die erfindungsgemäßen Zusammensetzungen sind flüssig.

Sehr vorteilhaft ist auch die relativ geringe Viskosität und damit eine einfache Handhabung der erfindungsgemäßen Zusammensetzungen.

In einer weiteren bevorzugten Ausführungsform der Erfindung weisen die erfindungsgemäßen Zusammensetzungen Viskositäten bei 35 °C von kleiner als 5.000 mPa·s, bevorzugt von 400 bis 2.000 mPa·s und besonders bevorzugt von 500 bis 1.000 mPa·s auf.

Bei dem für die Bestimmung der Viskosität verwendeten Gerät handelt es sich um ein Rotameter der Firma Thermo Haake (Viskotester 550). Als Spindel wurde MV-DIN (45,3 Umdrehungen / Minute) verwendet.

Die erfindungsgemäßen Zusammensetzungen enthalten keine organischen Lösungsmittel, wie beispielsweise niedere Alkohole, Diole oder andere Lösungsmittel.

In einer besonders bevorzugten Ausführungsform der Erfindung bestehen die erfindungsgemäßen Zusammensetzungen aus den Komponenten a), b), c) und d).

In einer weiteren besonders bevorzugten Ausführungsform der Erfindung bestehen die erfindungsgemäßen Zusammensetzungen aus den Komponenten a), b), c), d) und H₂NCH₂COO⁻ Q⁺, wobei Q⁺ für ein Kation ausgewählt aus Li⁺, Na⁺ und K⁺, vorzugsweise ausgewählt aus Na⁺ und K⁺ und besonders bevorzugt für Na⁺, steht. In diesen erfindungsgemäßen Zusammensetzung ist die Verbindung H₂NCH₂COO⁻ Q⁺, bezogen auf die gesamte erfindungsgemäße Zusammensetzung, vorzugsweise von 0,01 bis 1,0 und besonders bevorzugt von 0,05 bis 0,5 Gew.-%, enthalten.

Ein weiterer Gegenstand der Erfindung betrifft auch die Herstellung der erfindungsgemäßen Zusammensetzungen durch das erfindungsgemäße Verfahren.

Die erfindungsgemäßen Zusammensetzungen sind in vorteilhafter Weise zur Herstellung kosmetischer Zubereitungen geeignet.

Ein weiterer Gegenstand der Erfindung betrifft daher die Verwendung der erfindungsgemäßen Zusammensetzungen zur Herstellung einer kosmetischen Zubereitung. Besonders vorteilhaft dabei ist, dass die erfindungsgemäßen Zusammensetzungen wie aus dem erfindungsgemäßen Verfahren erhalten, d. h. ohne weitere Aufarbeitung oder Aufreinigung, verwendet werden können.

Die erfindungsgemäßen Zusammensetzungen sind zudem in vorteilhafter Weise als Tenside in kosmetischen Zubereitungen geeignet.

Ein weiterer Gegenstand der Erfindung betrifft daher die Verwendung der erfindungsgemäßen Zusammensetzungen als Tenside in kosmetischen Zubereitungen. Die erfindungsgemäßen Zusammensetzungen können auch hierbei direkt wie aus dem erfindungsgemäßen Verfahren erhalten verwendet werden.

Die nachfolgenden Beispiele und Anwendungen sollen die Erfindung näher erläutern, ohne sie jedoch darauf zu beschränken. Bei allen Prozentangaben handelt es sich um Gewichtsprozent (Gew.-%).

### Cocoylchlorid (A): Cocosschnitt mit reduziertem Anteil an C₁₆ und C₁₈ Spezifikation des verwendeten Cocoylchlorids:

| | |
|---|---|
| C₈/C₁₀: | 10,0 -14,0 % |
| C₁₂: | 60,0 - 62,0 % |
| C₁₄: | 19,0 - 24,0 % |
| C₁₆: | 3,0 - 10,0 % |
| C₁₈: | < 2,0 % |

### Beispiel 1

37,8 g (0,504 mol) Glycin wird in 276 g VE-Wasser (vollentsalztes Wasser) unter Rühren gelöst und der pH-Wert (telquel) mit Natronlauge (33 %) auf 12-13 eingestellt. Anschließend wird unter Rühren auf 30 - 35 °C aufgeheizt und 106,4 g (0,478 mol) Cocoylchlorid (A) innerhalb 6 Stunden bei 30 - 35 °C unter Kühlung des Reaktionsgemisches zudosiert. Der pH-Wert wird durch gleichzeitige Dosierung von Natronlauge (33 %) bei 12-13 gehalten. Gegen Ende der Dosierung des Cocoylchlorids lässt man den pH-Wert auf 9,5 - 10,5 abfallen. Zur Vervollständigung der Reaktion wird noch 2 Stunden bei pH 9,5 - 10,5 gerührt.

Das erhaltene Produkt hat folgende Eigenschaften: flüssig, opal, Trockenrückstand (1 h, 140 °C): 31,0 %, Glycinsalz (HPLC): 0,6 %, Fettsäuresalz (HPLC): 0,6 %, Viskosität (35 °C): 756 mPa·s, NaCl (Titration): 5,3 %, Aktivgehalt: 24,5 %

Die Berechnung der Gewichtsmenge an Acylglycinat in den erfindungsgemäßen Zusammensetzungen erfolgt durch die Formel "Gewichtsmenge an Acylglycinat = Trockenrückstand - Fettsäuresalz - Glycinsalz - Q⁺Cl⁻". Der Wert wird im Rahmen der vorliegenden Erfindung als "Aktivgehalt" bezeichnet.

### Vergleichsbeispiel 2 : höhere Reaktionstemperatur

37,8 g (0,504 mol) Glycin wird in 276 g VE-Wasser unter Rühren gelöst und der pH-Wert (telquel) mit Natronlauge (33 %) auf 12-13 eingestellt. Anschließend wird unter Rühren auf 45 - 50 °C aufgeheizt und 106,4 g (0,478 mol) Cocoylchlorid (A) innerhalb 6 Stunden bei 45 - 50 °C zudosiert. Der pH-Wert wird durch gleichzeitige Dosierung von Natronlauge (33 %) bei 12-13 gehalten. Nach ca. 1/6 der Dosierung des Cocoylchlorids wird der Ansatz gestoppt, da bereits die berechnete Menge an Natronlauge (33 %) des gesamten Ansatzes verbraucht ist.

Der Ansatz ist zweiphasig (Fettsäuresalz). Die erhöhte Temperatur führt vorwiegend zur Hydrolyse des Cocoylchlorids.

### Vergleichsbeispiel 3 : niedrigere Reaktionstemperatur

37,8 g (0,504 mol) Glycin wird in 276 g VE-Wasser unter Rühren gelöst und der pH-Wert (telquel) mit Natronlauge (33 %) auf 12-13 eingestellt. Anschließend wird unter Rühren auf 25 - 30 °C aufgeheizt und 106,4 g (0,478 mol) Cocoylchlorid (A) innerhalb 6 Stunden bei 25 - 30 °C unter Kühlung des Reaktionsgemisches zudosiert. Der pH-Wert wird durch gleichzeitige Dosierung von Natronlauge (33 %) bei 12-13 gehalten. Gegen Ende der Dosierung des Cocoylchlorids lässt man den pH-Wert auf 9,5 - 10,5 abfallen. Zur Vervollständigung der Reaktion wird noch 2 Stunden bei pH 9,5 -10,5 gerührt.

Das erhaltene Produkt hat folgende Eigenschaften: flüssig, trüb, Trockenrückstand (1 h, 140 °C): 30,9 %, Glycinsalz (HPLC): 1,3 %, Fettsäuresalz (HPLC): 2,4 %, Viskosität: nicht bestimmt, NaCl (Titration): 5,3%, Aktivgehalt: 21,9 %

### Beispiel 4 : höherer Glycinüberschuß

37,8 g (0,504 mol) Glycin wird in 250 g VE-Wasser unter Rühren gelöst und der pH-Wert (telquel) mit Natronlauge (33 %) auf 12-13 eingestellt. Anschließend wird unter Rühren auf 30 - 35 °C aufgeheizt und 95,2 g (0,428 mol) Cocoylchlorid (A) innerhalb 6 Stunden bei 30 - 35 °C unter Kühlung des Reaktionsgemisches zudosiert. Der pH-Wert wird durch gleichzeitige Dosierung von Natronlauge (33 %) bei 12-13 gehalten. Gegen Ende der Dosierung des Cocoylchlorids lässt man den pH-Wert auf 9,5 - 10,5 abfallen. Zur Vervollständigung der Reaktion wird noch 2 Stunden bei pH 9,5 - 10,5 gerührt.

Das erhaltene Produkt hat folgende Eigenschaften: flüssig, opal, Trockenrückstand (1 h, 140 °C): 31,3 %, Glycinsalz (HPLC): 1,3%, Fettsäuresalz (HPLC): 0,2 %, Viskosität (35 °C): 980 mPa·s, NaCl (Titration): 5,1 %, Aktivgehalt: 24,7 %

### Beispiel 5: höhere Konzentration

37,8 g (0,504 mol) Glycin wird in 246 g VE-Wasser unter Rühren gelöst und der pH-Wert (telquel) mit Natronlauge (33 %) auf 12-13 eingestellt. Anschließend wird unter Rühren auf 30 - 35 °C aufgeheizt und 106,4 g (0,478 mol) Cocoylchlorid (A) innerhalb 6 Stunden bei 30 - 35 °C unter Kühlung des Reaktionsgemisches zudosiert. Der pH-Wert wird durch gleichzeitige Dosierung von Natronlauge (33 %) bei 12-13 gehalten. Gegen Ende der Dosierung des Cocoylchlorids lässt man den pH-Wert auf 9,5 -10,5 abfallen. Zur Vervollständigung der Reaktion wird noch 2 Stunden bei pH 9,5 - 10,5 gerührt.

Das erhaltene Produkt hat folgende Eigenschaften: flüssig, opal, Trockenrückstand (1 h, 140 °C): 32,7 %, Glycinsalz (HPLC): 0,8 %, Fettsäuresalz (HPLC): 1,3 %, Viskosität (35 °C): 4.500 mPa·s, NaCl (Titration): 5,6 %, Aktivgehalt: 25,0 %

### Vergleichsbeispiel 6: salzfreie Herstellung in Propanol-2/Wasser

94,5 g (1,26 mol) Glycin wird in 400 g VE-Wasser und 276 g Propanol-2 unter Rühren gelöst und der pH-Wert (telquel) mit Natronlauge (33 %) auf 12-13 eingestellt. Anschließend werden unter Rühren bei 25 - 27 °C 283,9 g (1,24 mol) Cocoylchlorid (A) innerhalb 6 Stunden unter Kühlung des Reaktionsgemisches zudosiert. Der pH-Wert wird durch gleichzeitige Dosierung von Natronlauge (33 %) bei 12-13 gehalten. Zur Vervollständigung der Reaktion wird noch 2 Stunden bei pH 12-13 gerührt. Das erhaltene Produkt wird mit konz. Salzsäure auf pH 1 gestellt. Nach Abschalten des Rührers beginnt sofort die Phasentrennung. Die wässrige Phase wird abgetrennt und die organische Phase mit Natronlauge (33 %) auf pH 4,6 eingestellt. Nach Zugabe von 740 g Wasser wird mit Natronlauge (33 %) auf pH 7,0 gestellt. Anschließend wird bei vermindertem Druck (ca. 140-160 mbar) und 55 °C Propanol-2/Wasser abdestilliert bis der Propanol-2-Gehalt im Produkt unter 1 % gefallen ist. Mit Hilfe von Wasser und Natronlauge wird ein Feststoffgehalt von 30 % und ein pH-Wert von 10,0 eingestellt.

Das Produkt hat folgende Eigenschaften: flüssig, klar, Trockenrückstand (1 h, 140 °C): 30,3 %, Glycinsalz (HPLC): <0,1 %, Fettsäuresalz (HPLC): 1,0 %, Propanol-2 (GC): <0,1 %, Chlorid (Titration): 0,35 %, Viskosität: nicht bestimmt, NaCl (Titration): 0,6 %, Aktivgehalt: 28,7 % Vergleichsbeispiel 7: Cocosschnitt mit erhöhtem Anteil an C_{16/18}, hydriert Verteilung des verwendeten Cocoylchlorids (B) :

| | |
|---|---|
| C₁₂: | 55,6% |
| C₁₄: | 23,0% |
| C₁₆: | 11,1% |
| C₁₈ gesättigt: | 10,3% |

36,0 g (0,480 mol) Glycin wird in 280 g VE-Wasser unter Rühren gelöst und der pH-Wert (telquel) mit Natronlauge (33 %) auf 12-13 eingestellt. Anschließend wird unter Rühren auf 30 - 35 °C aufgeheizt und 109,6 g (0,456 mol) Cocoylchlorid (B) innerhalb 6 Stunden bei 30 - 35 °C unter Kühlung des Reaktionsgemisches zudosiert. Der pH-Wert wird durch gleichzeitige Dosierung von Natronlauge (33 %) bei 12-13 gehalten. Im Laufe der Dosierung des Cocoylchlorids wird der Ansatz immer zähflüssiger bis er nicht mehr rührbar ist. Durch Zugabe von 110 g Wasser und anschließende Erhöhung der Reaktionstemperatur auf 40 °C bleibt der Ansatz einigermaßen rührbar. Gegen Ende der Dosierung des Cocoylchlorids lässt man den den pH-Wert auf 9,5 - 10,5 abfallen. Zur Vervollständigung der Reaktion wird noch 2 Stunden bei pH 9,5 - 10,5 gerührt.

Das erhaltene Produkt hat folgende Eigenschaften: flüssig, trüb, Trockenrückstand (1 h, 140 °C): 25,8 %, Glycinsalz (HPLC): 0,8 %, Fettsäuresalz (HPLC): 1,2 %, Viskosität: nicht bestimmt, NaCl (Titration): 4,1 %, Aktivgehalt: 19,7 %

Die beschriebenen Beispiele belegen, dass hochkonzentrierte Alkalisalzglycinatlösungen mit > 22,0 %, vorzugsweise > 23,0 % und besonders bevorzugt > 24,0 % Aktivgehalt durch Verwendung eines C₁₈-armen Cocosfettsäureschnitts und Reaktion in Wasser ohne zusätzliche Lösungsmittel bei 30 - 35 °C erhalten werden können (Beispiel 1, Beispiel 4, Beispiel 5). In Beispiel 1 konnte eine Reinheit der Glycinatlösung von 97,6 % Acylglycinat bezogen auf die Summe aus Fettsäuresalz und Acylglycinat erreicht werden. Dagegen führen höhere Reaktionstemperaturen (Vergleichsbeispiel 2) zu stark erhöhten Fettsäuresalzgehalten. Niedrigere Reaktionstemperaturen (Vergleichsbeispiel 3) führen ebenfalls zu unerwünscht hohen Anteilen an Fettsäuresalzen. Bei Verwendung C₁₆/1C₁₈-reicher Fettsäurechloride (Vergleichsbeispiel 7) werden unerwünscht niedrige Aktivgehalte erhalten.

## Patentansprüche

1. Zusammensetzung enthaltend
a) ein oder mehrere Acylglycinate der Formel (I) 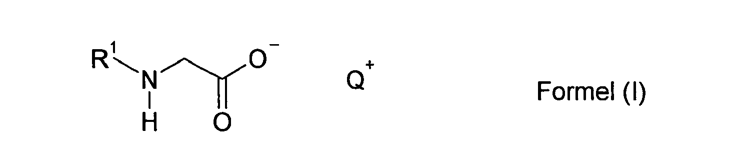 worin
R¹ für eine lineare oder verzweigte, gesättigte Alkanoylgruppe mit 6 bis 30, vorzugsweise 8 bis 22 und besonders bevorzugt 8 bis 18 Kohlenstoffatomen, oder für eine lineare oder verzweigte, ein- oder mehrfach ungesättigte Alkenoylgruppe mit 6 bis 30, vorzugsweise 8 bis 22 und besonders bevorzugt 12 bis 18 Kohlenstoffatomen, steht, und
Q⁺ für ein Kation ausgewählt aus den Alkalimetallkationen Li⁺, Na⁺ und K⁺, vorzugsweise ausgewählt aus Na⁺ und K⁺ und besonders bevorzugt für Na⁺, steht,
in Mengen von 21,0 - 28,0 Gew.-%, bezogen auf die gesamte Zusammensetzung,
und wobei der Anteil an Acylglycinaten der Formel (I) enthaltend Acylgruppen R¹ mit 18 oder mehr Kohlenstoffatomen, bezogen auf die Gesamtmenge an Acylglycinaten der Formel (I), kleiner als 2,0, bevorzugt kleiner als 1,8, Gew.-% ist,
b) ein oder mehrere Substanzen Q⁺Cl⁻, worin Q⁺ die Bedeutung von Q⁺ aus Formel (I) hat, in Mengen größer oder gleich 1,0 Gew.-%, bezogen auf die gesamte Zusammensetzung,
c) ein oder mehrere Fettsäuresalze der Formel (II) 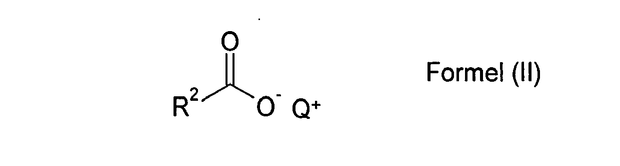 worin
R²CO die Bedeutung von R¹ aus Formel (I) und
Q⁺ die Bedeutung von Q⁺ aus Formel (I) hat,
in Mengen kleiner als 2,0 Gew.-%, vorzugsweise in Mengen größer als 0,01 Gew.-% und kleiner als 2,0 Gew.-% und besonders bevorzugt in Mengen größer als 0,1 Gew.-% und kleiner als 2,0 Gew.-%, bezogen auf die gesamte Zusammensetzung, und
d) Wasser,
e) aber keine organischen Lösungsmittel.

2. Zusammensetzung nach Anspruch 1, **dadurch gekennzeichnet, dass** sie als Komponente a) ein oder mehrere Acylglycinate der Formel (I), worin R¹ für eine Acylgruppe mit 8 bis 18 Kohlenstoffatomen steht, und Q⁺ für ein Kation ausgewählt aus Li⁺, Na⁺ und K⁺, vorzugsweise ausgewählt aus Na⁺ und K⁺ und besonders bevorzugt für Na⁺, steht, in Mengen von 21,0 - 28,0 Gew.-% und vorzugsweise in Mengen von 23,0 - 27,0 Gew.-%, bezogen auf die gesamte Zusammensetzung, enthält, und wobei der Anteil an Acylglycinaten der Formel (I) enthaltend Acylgruppen R¹ mit 18 Kohlenstoffatomen, bezogen auf die Gesamtmenge an Acylglycinaten der Formel (I), kleiner als 2,0, bevorzugt kleiner als 1,8, Gew.-% ist.

3. Zusammensetzung nach Anspruch 1 oder 2, **dadurch gekennzeichnet, dass** sie als Komponente a) Natriumcocoylglycinat mit C₈-₁₈-Acylgruppen in Mengen von 21,0 - 28,0 Gew.-% und vorzugsweise in Mengen von 23,0 - 27,0 Gew.-%, bezogen auf die gesamte Zusammensetzung, enthält, und wobei der Anteil an Acylglycinaten der Formel (I) mit C₁₈-Acylgruppen kleiner als 2,0, bevorzugt kleiner als 1,8, und besonders bevorzugt kleiner als 1,0 Gew.-%, bezogen auf die Gesamtmenge an Natriumcocoylglycinat, ist.

4. Zusammensetzung nach einem oder mehreren der Ansprüche 1 bis 3, **dadurch gekennzeichnet, dass** sie als Komponente a) Natriumcocoylglycinat mit C₈-₁₈-Acylgruppen in Mengen von 21,0 - 28,0 Gew.-% und vorzugsweise in Mengen von 23,0 - 27,0 Gew.-%, bezogen auf die gesamte Zusammensetzung, enthält, und wobei der Anteil an Acylglycinaten der Formel (I) mit C₈- und C₁₀-Acylgruppen zusammen größer als 5,0 Gew.-%, bevorzugt von 10,0 bis 14,0 Gew.-%, der Anteil an Acylglycinaten der Formel (I) mit C₁₂-Acylgruppen von 50,0 bis 72,0 Gew.-%, und der Anteil an Acylglycinaten der Formel (I) mit C₁₈-Acylgruppen kleiner als 2,0, bevorzugt kleiner als 1,8, und besonders bevorzugt kleiner als 1,0 Gew.-%, jeweils bezogen auf die Gesamtmenge an Natriumcocoylglycinat, ist.

5. Zusammensetzung nach Anspruch 1, **dadurch gekennzeichnet, dass** sie als Komponente a) ein oder mehrere Acylglycinate der Formel (I), worin R¹ für eine Acylgruppe mit 12 Kohlenstoffatomen steht, und Q⁺ für ein Kation ausgewählt aus Li⁺, Na⁺ und K⁺, vorzugsweise ausgewählt aus Na⁺ und K⁺ und besonders bevorzugt für Na⁺, steht, in Mengen von 21,0 - 28,0 Gew.-% und vorzugsweise in Mengen von 23,0 - 27,0 Gew.-%, bezogen auf die gesamte Zusammensetzung, enthält.

6. Zusammensetzung nach Anspruch 1, **dadurch gekennzeichnet, dass** sie als Komponente a) ein oder mehrere Acylglycinate der Formel (I), worin R¹ für eine Acylgruppe mit 12 Kohlenstoffatomen steht, und zusätzlich ein oder mehrere Acylglycinate der Formel (I), worin R¹ für eine Acylgruppe mit 14 Kohlenstoffatomen steht, und Q⁺ jeweils für ein Kation ausgewählt aus Li⁺, Na⁺ und K⁺, vorzugsweise ausgewählt aus Na⁺ und K⁺ und besonders bevorzugt für Na⁺, steht, zusammen in Mengen von 21,0 - 28,0 Gew.-% und vorzugsweise in Mengen von 23,0 - 27,0 Gew.-%, bezogen auf die gesamte Zusammensetzung, enthält.

7. Zusammensetzung nach einem oder mehreren der Ansprüche 1 bis 6, **dadurch gekennzeichnet, dass** sie, bezogen auf die gesamte Zusammensetzung, 1,0 bis 8,0, bevorzugt 2,0 bis 7,0 und besonders bevorzugt 4,0 bis 6,0 Gew.-% Q⁺Cl⁻ enthält.

8. Zusammensetzung nach einem oder mehreren der Ansprüche 1 bis 7, **dadurch gekennzeichnet, dass** sie, bezogen auf die gesamte Zusammensetzung, weniger als 1,8, bevorzugt weniger als 1,5 und besonders bevorzugt weniger als 1,0 Gew.-% an Fettsäuresalz der Formel (II) enthält.

9. Zusammensetzung nach einem oder mehreren der Ansprüche 1 bis 8, **dadurch gekennzeichnet, dass** sie eine Viskosität bei 35 °C von kleiner als 5.000 mPa·s, bevorzugt von 400 bis 2.000 mPa·s und besonders bevorzugt von 500 bis 1.000 mPa·s aufweist.

10. Verwendung einer Zusammensetzung nach einem oder mehreren der Ansprüche 1 bis 9 zur Herstellung einer kosmetischen Zubereitung.

11. Verwendung einer Zusammensetzung nach einem oder mehreren der Ansprüche 1 bis 9 als Tensid in einer kosmetischen Zubereitung.
